# FASCICULE DE BREVET EUROPEEN

(11) **EP 0 643 134 B1**
(45) Date de publication et mention de la délivrance du brevet: **05.12.2001**
(21) Numéro de dépôt: 94401813.4
(22) Date de dépôt: 05.08.1994
(51) Int. Cl.: C12N 15/31, C12N 15/74, C12N 1/21

(54) **Séquence d'acides nucléiques et plasmides comprenant au moins un mécanisme de résistance aux phages, bactéries les contenant et leur utilisation**
Nukleinsäuresequenzen und Plasmide welche mindestens einen Mechanismus für Phagenresistenz haben, Bakterien diese beinhaltend und ihre Verwendung
Nucleic acid sequences and plasmids comprising at least one mechanism of phage resistance, bacteria comprising said sequences and their utilisation

(30) Priorité: 09.08.1993 FR 9309777
(43) Date de publication de la demande: 15.03.1995
(73) Titulaire: SKW Nature Products Holding France S.A.S., 92100 Boulogne-Billancourt (FR)
(72) Inventeur: Prevots, Fabien, F-31400 Toulouse (FR); Remy, Elisabeth, F-31000 Toulouse (FR); Ritzenthaler, Paul, F-31320 Castanet Tolosan (FR)
(74) Mandataire: Gillard, Marie-Louise

(56) Documents cités:
- EP-A- 0 452 224
- WO-A-92/05260
- APPLIED AND ENVIRONMENTAL MICROBIOLOGY, vol.47, no.5, Mai 1984 pages 979 - 985 SANDERS, M. E. ET AL. 'Phage resistance in a phage-insensitive strain of Streptococcus lactis: Temperature-dependent phage development and host-controlled phage replication'

### LISTE DE SEQUENCES

(1) INFORMATION GENERALE:
   (i) DEPOSANT:
      (A) NOM: SKW NATURE PRODUCTS HOLDING FRANCE S.A.S.
      (B) RUE: 4, place des Ailes
      (C) VILLE: BOULOGNE-BILLANCOURT
      (E) PAYS: FRANCE
      (F) CODE POSTAL: 92100
      (G) TELEPHONE : 01.47.12.25.25
      (H) TELECOPIE : 01.47.12.26.56
   (ii) TITRE DE L' INVENTION: SEQUENCE D'ACIDES NUCLEIQUES ET PLASMIDES COMPRENANT AU MOINS UN MECANISME DE RESISTANCE AUX PHAGES, BACTERIES LES CONTENANT ET LEUR UTILISATION
   (iii) NOMBRE DE SEQUENCES: 7
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 1875 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Lactococcus lactis
   (ix) CARACTERISTIQUE ADDITIONELLE:
      (A) NOM/CLE: CDS
      (B) EMPLACEMENT: 202..1821
   (xi) DESCRIPTION DE LA SEQUENCE: SEQ ID NO: 1:
   GAACATAGAA TAGATTACGG GTTAATGGAC AATAAATGCA AACGATTTTG AGAAATTTAA     60
   TAAGAAAAGA AGAGTGTCCA GAAAATGACC ATTTTCTGAA CGCCATATTA AAAATTTTTT    120
   GATAATTCCC AATATATTAT AATATAGCTT CAATGTTAAA ATTTATATGA TATAATATAA    180
   GAAAATTTTT AAAAAAATAG A ATG GAT ATA ATA ATG GAC TTT AAA ACT ATG     231
                           Met Asp Ile Ile Met Asp Phe Lys Thr Met
                            1               5                  10
   TTA AGC TAT CTT GTA AGT CAA GAT GAT GAA ATT TCT TTA AGA AAT GAT     279
   Leu Ser Tyr Leu Val Ser Gln Asp Asp Glu Ile Ser Leu Arg Asn Asp
                    15                  20                  25
   ATT AAA CAT GAA GAA GTA TAT AAA ATT TTA GAG AAT AAG TTT GCT TCT     327
   Ile Lys His Glu Glu Val Tyr Lys Ile Leu Glu Asn Lys Phe Ala Ser
                30                  35                  40
   ATA ATG CCG AAG TTT AAA ACA AAA GGT TAT AAG TTT AAA GAT ACT ACT     375
   Ile Met Pro Lys Phe Lys Thr Lys Gly Tyr Lys Phe Lys Asp Thr Thr
            45                  50                  55
   GAA GTT TTG ACA TTC GCT AAA TTT GTA TTT TTG CTA CAA GAG TGG GGG     423
   Glu Val Leu Thr Phe Ala Lys Phe Val Phe Leu Leu Gln Glu Trp Gly
        60                  65                  70
   TTG AAG GAT ATA CAG TTT TAT AAG AAC ACT AAT AGT TTC TTA TTT GGA     471
   Leu Lys Asp Ile Gln Phe Tyr Lys Asn Thr Asn Ser Phe Leu Phe Gly
    75                  80                  85                  90
   TAT ATT ATA CCG CAA ATT AAT AAA GAA TTT GAT TTA TTG AGA TTT GGG     519
   Tyr Ile Ile Pro Gln Ile Asn Lys Glu Phe Asp Leu Leu Arg Phe Gly
                    95                 100                 105
   GAA AAT TAC AAT ATT AGT ATA GAA CTC AAA AGT AAA ACA ACA GTA GAA     567
   Glu Asn Tyr Asn Ile Ser Ile Glu Leu Lys Ser Lys Thr Thr Val Glu
               110                 115                 120
   GCA CAA AAG CAA CAA CTT TGT AAG AAC TAT TTT TAC CTA AAT TTT TTA    615
   Ala Gln Lys Gln Gln Leu Cys Lys Asn Tyr Phe Tyr Leu Asn Phe Leu
           125                 130                 135
   TCA ACT AAA ACT AGG TAT ATT AGT ATA TCC CCA GAT ATA TCT AGT TAC    663
   Ser Thr Lys Thr Arg Tyr Ile Ser Ile Ser Pro Asp Ile Ser Ser Tyr
       140                 145                 150
   ATA GAA TAT ATT CCA AGT GAA AAT AAG TAT ATC AAT TTA AGT GGA ACT    711
   Ile Glu Tyr Ile Pro Ser Glu Asn Lys Tyr Ile Asn Leu Ser Gly Thr
   155                 160                 165                 170
   GAA ATT TGT GAT ATT ATT ATT AAA CAA GAG TTT TTA GAG TAT AAT ACA    759
   Glu Ile Cys Asp Ile Ile Ile Lys Gln Glu Phe Leu Glu Tyr Asn Thr
                   175                 180                 185
   AAA GAG GTT GAT AGT TTT TTT GAT ATA AAA AAT TAT TTA GTT TCT CCT    807
   Lys Glu Val Asp Ser Phe Phe Asp Ile Lys Asn Tyr Leu Val Ser Pro
               190                 195                 200
   TTC AAT GAT GTT GAA AAA TTT CTT GAT GAT AAA TAT TTT TTA ACA CCT    855
   Phe Asn Asp Val Glu Lys Phe Leu Asp Asp Lys Tyr Phe Leu Thr Pro
           205                 210                 215
   CAC CAA GAC CAG ATT GTT AAA GAA ATT ACT GAA CCA AGT GAC AAA AAA    903
   His Gln Asp Gln Ile Val Lys Glu Ile Thr Glu Pro Ser Asp Lys Lys
       220                 225                 230
   ACT TTT GGT ATA AAA GGA AAT CCA GGA ACA GGA AAA TCT TTG CTA GTT    951
   Thr Phe Gly Ile Lys Gly Asn Pro Gly Thr Gly Lys Ser Leu Leu Val
   235                 240                 245                 250
   TAC CAT ATA TGT AAA AAA TTA ATG GAG AAA AAT AAA AGA GTT GCT ATA    999
   Tyr His Ile Cys Lys Lys Leu Met Glu Lys Asn Lys Arg Val Ala Ile
                   255                 260                 265
   GTT CAT GGA GCA AAT CTA AAT AAT GGT CAA CAA AGA TTA GCT CTG CGT   1047
   Val His Gly Ala Asn Leu Asn Asn Gly Gln Gln Arg Leu Ala Leu Arg
               270                 275                 280
   GGT TTC ACA ATT TTT CCT GTT AAA TCG ATC ATA GAG GTA TTA GAT AAT   1095
   Gly Phe Thr Ile Phe Pro Val Lys Ser Ile Ile Glu Val Leu Asp Asn
           285                 290                 295
   GCA GAC AAA TAC GAT TAC ATT GTT GTT GAC GAA GCT CAA CGT CTA AGA      1143
   Ala Asp Lys Tyr Asp Tyr Ile Val Val Asp Glu Ala Gln Arg Leu Arg
       300                 305                 310
   CAA GAC TTA GGA GAA CAA TAT ACT AAA TTG GTT GAT ACT ATT GAA AAT      1191
   Gln Asp Leu Gly Glu Gln Tyr Thr Lys Leu Val Asp Thr Ile Glu Asn
   315                 320                 325                 330
   TCT CAA ACA AAA TTT ATT ATC TCA CTA GAT GGA AGA CAA ACT TTG AAT      1239
   Ser Gln Thr Lys Phe Ile Ile Ser Leu Asp Gly Arg Gln Thr Leu Asn
                   335                 340                 345
   AAA TAT GAA ATA GAA GAA AAT TCC ATA AAA TTA TTT AAA TAT ATA AAA      1287
   Lys Tyr Glu Ile Glu Glu Asn Ser Ile Lys Leu Phe Lys Tyr Ile Lys
               350                 355                 360
   AAT AAA GGA GTA ACT TTT AGT CTT AAA GAT AAG TTT AGA ACT AAC CCA      1335
   Asn Lys Gly Val Thr Phe Ser Leu Lys Asp Lys Phe Arg Thr Asn Pro
           365                 370                 375
   GAA ATG AGC AAA TTT ATC CAA CTT CTA TTC AAA ATA CCC ATG TAT AAA      1383
   Glu Met Ser Lys Phe Ile Gln Leu Leu Phe Lys Ile Pro Met Tyr Lys
       380                 385                 390
   AAA ATA GAT TTA ATT TCA AAC ATA GAT CAT AAT ATT ATA ATT AAA TAT      1431
   Lys Ile Asp Leu Ile Ser Asn Ile Asp His Asn Ile Ile Ile Lys Tyr
   395                 400                 405                 410
   TTT GAT AAC AGA GAA TCG GGA AAT GAA TAT ATT TCC GAT ATG GAT TCA      1479
   Phe Asp Asn Arg Glu Ser Gly Asn Glu Tyr Ile Ser Asp Met Asp Ser
                   415                 420                 425
   AAC TCA GAT TGG GAA GTA CTT AAT TAC ACG AAG GAT AGA TTT AGG AAA      1527
   Asn Ser Asp Trp Glu Val Leu Asn Tyr Thr Lys Asp Arg Phe Arg Lys
               430                 435                 440
   ACA GGA ATT GGT AAA ATG TGT GGT AAT GGT TTA ACA TCA CAT AGT ATT      1575
   Thr Gly Ile Gly Lys Met Cys Gly Asn Gly Leu Thr Ser His Ser Ile
           445                 450                 455
   ATC GGT CAA GAA TTT GAT AAA GTT ATT ATA CCT TTG GAT TCG AAT TTT      1623
   Ile Gly Gln Glu Phe Asp Lys Val Ile Ile Pro Leu Asp Ser Asn Phe
       460                 465                 470
   TTT TAT AAA GAA CAA AAA ATA ATT GAT AGT AAA ACG GGT GAA AGT AAA        1671
   Phe Tyr Lys Glu Gln Lys Ile Ile Asp Ser Lys Thr Gly Glu Ser Lys
   475                 480                 485                 490
   GTT TTT AAA TTA TTG GAA ACG ACT GAT AAT TTT TAC CCA CTT GAA AAA        1719
   Val Phe Lys Leu Leu Glu Thr Thr Asp Asn Phe Tyr Pro Leu Glu Lys
                   495                 500                 505
   ATG TTA TAT CAA AAT CTT ACT CGC ACA AGG GGA AAA ATA GAA TTT GTA        1767
   Met Leu Tyr Gln Asn Leu Thr Arg Thr Arg Gly Lys Ile Glu Phe Val
               510                 515                 520
   ATT ATT GGA AAT CGT TCA ATT TTT AAT GAA ATA TGT GGA TTG CTA GAT        1815
   Ile Ile Gly Asn Arg Ser Ile Phe Asn Glu Ile Cys Gly Leu Leu Asp
           525                 530                 535
   AGT TTA TAAAGTTCTG TCTCAAAGTT AAAAAAAGTG AAATCACTCG CAACAGAACA         1871
   Ser Leu
       540
   GTTT                                                                   1875
(2) INFORMATION POUR LA SEQ ID NO: 2:
   (i) CARACTERISTIQUES DE LA SEQUENCE:
      (A) LONGUEUR: 540 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Asp Ile Ile Met Asp Phe Lys Thr Met Leu Ser Tyr Leu Val Ser
    1               5                  10                  15
   Gln Asp Asp Glu Ile Ser Leu Arg Asn Asp Ile Lys His Glu Glu Val
               20                  25                  30
   Tyr Lys Ile Leu Glu Asn Lys Phe Ala Ser Ile Met Pro Lys Phe Lys
           35                  40                  45
   Thr Lys Gly Tyr Lys Phe Lys Asp Thr Thr Glu Val Leu Thr Phe Ala
       50                  55                  60
   Lys Phe Val Phe Leu Leu Gln Glu Trp Gly Leu Lys Asp Ile Gln Phe
   65                  70                  75                  80
   Tyr Lys Asn Thr Asn Ser Phe Leu Phe Gly Tyr Ile Ile Pro Gln Ile
   85 90 95
   Asn Lys Glu Phe Asp Leu Leu Arg Phe Gly Glu Asn Tyr Asn Ile Ser
   100 105 110
   Ile Glu Leu Lys Ser Lys Thr Thr Val Glu Ala Gln Lys Gln Gln Leu
   115 120 125
   Cys Lys Asn Tyr Phe Tyr Leu Asn Phe Leu Ser Thr Lys Thr Arg Tyr
   130 135 140
   Ile Ser Ile Ser Pro Asp Ile Ser Ser Tyr Ile Glu Tyr Ile Pro Ser
   145 150 155 160
   Glu Asn Lys Tyr Ile Asn Leu Ser Gly Thr Glu Ile Cys Asp Ile Ile
   165 170 175
   Ile Lys Gln Glu Phe Leu Glu Tyr Asn Thr Lys Glu Val Asp Ser Phe
   180 185 190
   Phe Asp Ile Lys Asn Tyr Leu Val Ser Pro Phe Asn Asp Val Glu Lys
   195 200 205
   Phe Leu Asp Asp Lys Tyr Phe Leu Thr Pro His Gln Asp Gln Ile Val
   210 215 220
   Lys Glu Ile Thr Glu Pro Ser Asp Lys Lys Thr Phe Gly Ile Lys Gly
   225 230 235 240
   Asn Pro Gly Thr Gly Lys Ser Leu Leu Val Tyr His Ile Cys Lys Lys
   245 250 255
   Leu Met Glu Lys Asn Lys Arg Val Ala Ile Val His Gly Ala Asn Leu
   260 265 270
   Asn Asn Gly Gln Gln Arg Leu Ala Leu Arg Gly Phe Thr Ile Phe Pro
   275 280 285
   Val Lys Ser Ile Ile Glu Val Leu Asp Asn Ala Asp Lys Tyr Asp Tyr
   290 295 300
   Ile Val Val Asp Glu Ala Gln Arg Leu Arg Gln Asp Leu Gly Glu Gln
   305 310 315 320
   Tyr Thr Lys Leu Val Asp Thr Ile Glu Asn Ser Gln Thr Lys Phe Ile
   325 330 335
   Ile Ser Leu Asp Gly Arg Gln Thr Leu Asn Lys Tyr Glu Ile Glu Glu
   340 345 350
   Asn Ser Ile Lys Leu Phe Lys Tyr Ile Lys Asn Lys Gly Val Thr Phe
   355 360 365
   Ser Leu Lys Asp Lys Phe Arg Thr Asn Pro Glu Met Ser Lys Phe Ile
   370 375 380
   Gln Leu Leu Phe Lys Ile Pro Met Tyr Lys Lys Ile Asp Leu Ile Ser
   385                 390                 395                 400
   Asn Ile Asp His Asn Ile Ile Ile Lys Tyr Phe Asp Asn Arg Glu Ser
                   405                 410                 415
   Gly Asn Glu Tyr Ile Ser Asp Met Asp Ser Asn Ser Asp Trp Glu Val
               420                 425                 430
   Leu Asn Tyr Thr Lys Asp Arg Phe Arg Lys Thr Gly Ile Gly Lys Met
           435                 440                 445
   Cys Gly Asn Gly Leu Thr Ser His Ser Ile Ile Gly Gln Glu Phe Asp
       450                 455                 460
   Lys Val Ile Ile Pro Leu Asp Ser Asn Phe Phe Tyr Lys Glu Gln Lys
   465                 470                 475                 480
   Ile Ile Asp Ser Lys Thr Gly Glu Ser Lys Val Phe Lys Leu Leu Glu
                   485                 490                 495
   Thr Thr Asp Asn Phe Tyr Pro Leu Glu Lys Met Leu Tyr Gln Asn Leu
               500                 505                 510
   Thr Arg Thr Arg Gly Lys Ile Glu Phe Val Ile Ile Gly Asn Arg Ser
           515                 520                 525
   Ile Phe Asn Glu Ile Cys Gly Leu Leu Asp Ser Leu
       530                 535                 540
(2) INFORMATION POUR LA SEQ ID NO: 3:
   (i) CARACTERISTIQUES DE LA SEQUENCE:
      (A) LONGUEUR: 28 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (ix) CARACTERISTIQUE ADDITIONELLE:
      (A) NOM/CLE: misc_signal
      (B) EMPLACEMENT: 3..8
      (D) AUTRES RENSEIGNEMENTS: /function= "SITE DE RESTRICTION EcoRI"
   (ix) CARACTERISTIQUE ADDITIONELLE:
      (A) NOM/CLE: misc_structure
      (B) EMPLACEMENT: 9..28
      (D) AUTRES RENSEIGNEMENTS: /function= "SEQ HOMOLOGUE DES NUCLEOTIDES 1-20 DE SEQ.ID NO.1"
   (xi) DESCRIPTION DE LA SEQUENCE: SEQ ID NO: 3:
   GGGAATTCGA ACATAGAATA GATTACGG 28
(2) INFORMATION POUR LA SEQ ID NO: 4:
   (i) CARACTERISTIQUES DE LA SEQUENCE:
      (A) LONGUEUR: 28 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (ix) CARACTERISTIQUE ADDITIONELLE:
      (A) NOM/CLE: misc_signal
      (B) EMPLACEMENT: 3..8
      (D) AUTRES RENSEIGNEMENTS: /function= "SITE DE RESTRICTION BamHI"
   (ix) CARACTERISTIQUE ADDITIONELLE:
      (A) NOM/CLE: misc_structure
      (B) EMPLACEMENT: 9..28
      (D) AUTRES RENSEIGNEMENTS: /function= "SEQ.HOMOLOGUE ADNc CORRESP.NUCLEOT. 1856-1875 de SEQ.ID No.1"
   (xi) DESCRIPTION DE LA SEQUENCE: SEQ ID NO: 4:
   GGGGATCCAA ACTGTTCTGT TGCGAGTG 28
(2) INFORMATION POUR LA SEQ ID NO: 5:
   (i) CARACTERISTIQUES DE LA SEQUENCE:
      (A) LONGUEUR: 28 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (ix) CARACTERISTIQUE ADDITIONELLE:
      (A) NOM/CLE: misc_signal
      (B) EMPLACEMENT: 3..8
      (D) AUTRES RENSEIGNEMENTS: /function= "SITE DE RESTRICTION EcoRI"
   (ix) CARACTERISTIQUE ADDITIONELLE:
      (A) NOM/CLE: misc_structure
      (B) EMPLACEMENT: 9..28
      (D) AUTRES RENSEIGNEMENTS: /function= "SEQ.HOMOLOGUE NUCLEOTIDES 1292-1311 de SEQ. No. 1"
   (xi) DESCRIPTION DE LA SEQUENCE: SEQ ID NO: 5:
   GGGAATTCAA GGAGTAACTT TTAGTCTT 28
(2) INFORMATION POUR LA SEQ ID NO: 6:
   (i) CARACTERISTIQUES DE LA SEQUENCE:
      (A) LONGUEUR: 28 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (ix) CARACTERISTIQUE ADDITIONELLE:
      (A) NOM/CLE: misc_signal
      (B) EMPLACEMENT: 3..8
      (D) AUTRES RENSEIGNEMENTS: /function= "SITE DE RESTRICTION EcoRI"
   (ix) CARACTERISTIQUE ADDITIONELLE:
      (A) NOM/CLE: misc_structure
      (B) EMPLACEMENT: 9..28
      (D) AUTRES RENSEIGNEMENTS: /function= "SEQ.HOMOLOGUE ADNc CORRESP. NUCLEOT.1773-1792 SEQ.ID No.1"
   (xi) DESCRIPTION DE LA SEQUENCE: SEQ ID NO: 6:
   GGGAATTCTA AAAATTGAAC GATTTCCA 28
(2) INFORMATION POUR LA SEQ ID NO: 7:
   (i) CARACTERISTIQUES DE LA SEQUENCE:
      (A) LONGUEUR: 3234 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Lactococcus lactis
   (xi) DESCRIPTION DE LA SEQUENCE: SEQ ID NO: 7:
   AAGCTTTACG TCTTGCTTTG AAAATTTCTC AGGTCTTCCT CCAAGTCGTC CTCTTGCACG    60
   TGCAGCTTCT CTACCAGCGG CAGAACGCTC CAAAATAAGA TTTCGCTCAA ATTCCGCAAA   120
   AGCCGCAAAC AAATGAAACA TCAATTGTCC AGTCGAACTT GATTTATCCA TTGTAATATT   180
   TTCTTGCAAG CTATGGAAAC TTACTCCTTT ATCATTAAGT GAATTAACTA TGCTAATTAA   240
   GTCCTCCATA TTTCTTCCTA ATCTATCTAA CCGCCAAACA ACAATTGTAT CTCCAGAACG   300
   AGAAAATTCG ATGGCGGATT TTAAACCAGG TCTTTCTTTT TTACTTCCTG ACATATGGTC   360
   AGTAAATATT TTTTCACAGT TATATTTTTT GAGACTATCT TTTTGTAAAT CCAAATTTTG   420
   AAGTCCAGTT GAAACTCGTG CGTATCCTAT ATTCATTTTT TTCTCCTTCA TTTTAATTTA   480
   TTGTATCATA ACTTAAAAAT ATATGTATAA ATGAACATAG AATAGATTAC GGGTTAATGG   540
   ACAATAAATG CAAACGATTT TGAGAAATTT AATAAGAAAA GAAGAGTGTC CAGAAAATGA   600
   CCATTTTCTG AACGCCATAT TAAAAATTTT TTGATAATTC CCAATATATT ATAATATAGC   660
   TTCAATGTTA AAATTTATAT GATATAATAT AAGAAAATTT TTAAAAAAAT AGAATGGATA   720
   TAATAATGGA CTTTAAAACT ATGTTAAGCT ATCTTGTAAG TCAAGATGAT GAAATTTCTT 780
   TAAGAAATGA TATTAAACAT GAAGAAGTAT ATAAAATTTT AGAGAATAAG TTTGCTTCTA 840
   TAATGCCGAA GTTTAAAACA AAAGGTTATA AGTTTAAAGA TACTACTGAA GTTTTGACAT 900
   TCGCTAAATT TGTATTTTTG CTACAAGAGT GGGGGTTGAA GGATATACAG TTTTATAAGA 960
   ACACTAATAG TTTCTTATTT GGATATATTA TACCGCAAAT TAATAAAGAA TTTGATTTAT 1020
   TGAGATTTGG GGAAAATTAC AATATTAGTA TAGAACTCAA AAGTAAAACA ACAGTAGAAG 1080
   CACAAAAGCA ACAACTTTGT AAGAACTATT TTTACCTAAA TTTTTTATCA ACTAAAACTA 1140
   GGTATATTAG TATATCCCCA GATATATCTA GTTACATAGA ATATATTCCA AGTGAAAATA 1200
   AGTATATCAA TTTAAGTGGA ACTGAAATTT GTGATATTAT TATTAAACAA GAGTTTTTAG 1260
   AGTATAATAC AAAAGAGGTT GATAGTTTTT TTGATATAAA AAATTATTTA GTTTCTCCTT 1320
   TCAATGATGT TGAAAAATTT CTTGATGATA AATATTTTTT AACACCTCAC CAAGACCAGA 1380
   TTGTTAAAGA AATTACTGAA CCAAGTGACA AAAAAACTTT TGGTATAAAA GGAAATCCAG 1440
   GAACAGGAAA ATCTTTGCTA GTTTACCATA TATGTAAAAA ATTAATGGAG AAAAATAAAA 1500
   GAGTTGCTAT AGTTCATGGA GCAAATCTAA ATAATGGTCA ACAAAGATTA GCTCTGCGTG 1560
   GTTTCACAAT TTTTCCTGTT AAATCGATCA TAGAGGTATT AGATAATGCA GACAAATACG 1620
   ATTACATTGT TGTTGACGAA GCTCAACGTC TAAGACAAGA CTTAGGAGAA CAATATACTA 1680
   AATTGGTTGA TACTATTGAA AATTCTCAAA CAAAATTTAT TATCTCACTA GATGGAAGAC 1740
   AAACTTTGAA TAAATATGAA ATAGAAGAAA ATTCCATAAA ATTATTTAAA TATATAAAAA 1800
   ATAAAGGAGT AACTTTTAGT CTTAAAGATA AGTTTAGAAC TAACCCAGAA ATGAGCAAAT 1860
   TTATCCAACT TCTATTCAAA ATACCCATGT ATAAAAAAAT AGATTTAATT TCAAACATAG 1920
   ATCATAATAT TATAATTAAA TATTTTGATA ACAGAGAATC GGGAAATGAA TATATTTCCG 1980
   ATATGGATTC AAACTCAGAT TGGGAAGTAC TTAATTACAC GAAGGATAGA TTTAGGAAAA 2040
   CAGGAATTGG TAAAATGTGT GGTAATGGTT TAACATCACA TAGTATTATC GGTCAAGAAT 2100
   TTGATAAAGT TATTATACCT TTGGATTCGA ATTTTTTTTA TAAAGAACAA AAAATAATTG 2160
   ATAGTAAAAC GGGTGAAAGT AAAGTTTTTA AATTATTGGA AACGACTGAT AATTTTTACC 2220
   CACTTGAAAA AATGTTATAT CAAAATCTTA CTCGCACAAG GGGAAAAATA GAATTTGTAA 2280
   TTATTGGAAA TCGTTCAATT TTTAATGAAA TATGTGGATT GCTAGATAGT TTATAAAGTT 2340
   CTGTCTCAAA GTTAAAAAAA GTGAAATCAC TCGCAACAGA ACAGTTTGAC ATTAAGTCCA 2400
   TTTCTTATAC CCAAAAATGT ATAATTCTAA TCTATTTATT TTAGGAAATT ATTTTTTCAA 2460
   AATGATTTGG AGTGAGATAC CCCAAACTTT GATGGATTCT TTTAAATAAA ATTTCAAAGC 2520
   GCTCACTCCA GAAATGCTAA GTTTCGGAAA AAATTTGAAT TTTTCGTAAA GATATTATTT 2580
   TTGGAGTGAA AATCATAAAA TTCTTCTTTT AAAAACTTCC GCAAGTTTTT TAAGGAAAAT 2640
   AGTTACTTAC GTCCAAACTC AAAAAATTTT TATAAAATTG TAGTTCATTT GACGGTAAGT 2700
   CTTATTATTT AATGATACCT AGTAGTTAAT AATTTGATTA TATTTGTAAT TACAGATATA 2760
   ATCAAATTAT TTGGAGGTAT TAATAGTATG GAAAGTAAGT TTAACGGAGA TGAGTCTGGA 2820
   TATTATGATA ATAAAGATAA TTTTTATATT AATGGCTCTT TAAAATATAA AGACGATATG 2880
   GAAGTTGGTC CAATTTTACA GCATGAGCAT GGACATTGGT TTATTTTTAT GACATCTTCA 2940
   CTAGGGCTCT TAATTCGTAT GTGTTCAAAA ATATCAATAA CAGACAATAG TAAAGATTTA 3000
   ATTTTGGAGG GATTAAGTAA GTATTATAGA AGAATGAATG AGGAAGTTGC TACATATACT   3060
   GAGATGATAA CATATCTTAT GATGAATGGT AGAGAACAAT TTCTTCGCAA AGTTGATTAT   3120
   CTAAAATATA ATAATAAGTC ATACTATAAA TACTATAAAA AATTATCTTG TAGAAATATT   3180
   TTATTGAGTC AGTCAATGAT TTTAACTTAT GATAAAGAAA AACTTAAAAA GCTT         3234

## Description

La présente invention a pour objet une nouvelle séquence d'acides nucléiques et des plasmides susceptibles de s'hybrider avec celle-ci, porteurs d'au moins un mécanisme de résistance aux phages, les bactéries lactiques contenant cette séquence ou ces plasmides, en particulier les lactocoques appartenant à l'espèce Lactococcus lactis, l'utilisation de certaines souches de ces lactocoques pour transférer, notamment par conjugaison, un mécanisme de résistance aux phages à des souches d'intérêt industriel, en particulier dans l'industrie laitière, et à l'emploi de certaines souches Lactococcus lactis pour obtenir ces plasmides.

Les bactéries lactiques sont impliquées dans l'élaboration et la conservation d'un grand nombre de produits alimentaires, tels que les fromages, le beurre, les yaourts, le saucisson ou la choucroute. Parmi ceux-ci les produits laitiers occupent une place particulièrement importante. La transformation industrielle du lait est faite dans des cuves de fermentation de plus en plus grandes, dans lesquelles l'apparition de phages des bactéries lactiques peut avoir des conséquences graves, voire catastrophiques : variation des caractéristiques, notamment organoleptiques, du produit final ; perte du produit présent dans la cuve et nécessité de décontaminer cette dernière ainsi que les installations environnantes. Il existe donc dans l'industrie laitière un besoin impérieux de nouveaux moyens et de nouvelles méthodes permettant de rendre les bactéries lactiques plus résistantes aux phages.

Les phages des bactéries lactiques appartiennent à trois grands groupes d'homologie (I), (II) et (III) définis par des études d'hybridation ADN/ADN selon RELANO P. et al. (1987), J. Gen. Microbiol. 133, 3053-3063. Les groupes (I) et (III) comprennent uniquement des phages virulents. Le groupe (II) comprend des phages virulents et des phages tempérés. A l'intérieur d'un même groupe les homologies sont fortes et, d'un groupe à l'autre, les homologies sont très faibles. Les phages du groupe (I) ont une nucléocapside oblongue alors que les phages des groupes (II) et (III) ont une nucléocapside isométrique.

On sait qu'il existe plusieurs mécanismes de résistance aux phages dont les trois principaux sont :
- l'inhibition de l'adsorption des phages ; dans ce mécanisme, l'adsorption du phage par la bactérie est inhibée ou retardée.
- le système restriction/modifcation ; ce système fait intervenir une enzyme de restriction qui dégrade l'ADN du phage dès son entrée dans la bactérie.
- l'infection abortive ; selon ce troisième mécanisme, l'adsorption des phages est normale, mais leur multiplication ne se produit pas.

Ces mécanismes sont décrits en détail par SANDERS M. dans Biochimie 70, (1988), 411-421.

De nombreuses études ont déjà été effectuées pour mettre au point des bactéries lactiques résistantes aux phages.

A cet effet, on peut se référer en particulier aux articles ci-après :
- VLEGELS et al. ; Neth. Milk and Dairy J. 43 (1989) 245-259
- SANDERS and KLAENHAMMER. Applied and Environ. Microbiol. (1983) vol. 46, 1125-1133
   qui concernent des plasmides qui inhibent l'adsorption des phages ;
- Audrey W. JARVIS ; Applied and Environ. Microbiol.; March 1988 p. 777-783 ;
- EP-A3-0208 468 ;
- COFFEY et al. ; Neth. Milk and Dairy J. 43 (1989) 229-244 ;
- KLAENHAMMER and SANOZKY; Journal of General Microbiology (1985), 131, 1531-1541
   qui décrivent des plasmides qui confèrent une résistance aux phages par le mécanisme d'infection abortive.
- JOSEPHSEN and KLAENHAMMER, Plasmid 23, 71-75 (1990)
- brevet US 4 883 756
- GAUTIER and CHOPIN; Applied and Environ. Microbiology (1987) 53 p. 923-927,
ces derniers articles décrivent notamment des plasmides conférant la résistance aux phages par le mécanisme de restriction/modification.

Les demanderesses ont également effectué des travaux dans ce domaine et décrit dans EP-A1-452 224 une molécule d'ADN comprenant au moins un mécanisme de résistance aux phages, qui comporte une partie fonctionnelle du fragment HindIII-HindIII d'environ 3,3 kb du plasmide pPF 144-1 présent dans la souche Escherichia coli déposée le 9 avril 1991 à la Collection Nationale de Culture de Microorganismes (CNCM) de l'Institut Pasteur, Paris, sous le N°I-1070.

Ce fragment HindIII-HindIII d'environ 3,3kb, a été isolé à partir du plasmide pPF 144 contenu dans la souche Lactococcus lactis ssp lactis, déposée à la C.N.C.M. sous le N°I-945, laquelle est un transconjugant issu du croisement de la souche donneuse Lactococcus lactis ssp lactis S91 déposée à la C.N.C.M. le 12 avril 1991 sous le N°I-940 et de la souche Lactococcus lactis ssp lactis S45, dérivée de la souche Lactococcus lactis ssp lactis C2-LL.Mc.Kay et al, 1977, J. Bacteriol.257-265. Ce fragment est porteur d'un ou plusieurs mécanismes de résistance aux phages.

Poursuivant leurs travaux, elles ont isolé à partir de cette séquence d'ADN HindIII-HindIII de 3,3 kb, une séquence d'ADN de 1,9 kb qui confère à elle seule la résistance aux phages.

La présente invention a donc pour objet une nouvelle séquence d'acides nucléiques comprenant au moins un mécanisme de résistance aux phages, ladite séquence ayant environ 1,9 kb et étant constituée par :
a) la séquence d'ADN présentant l'enchaînement d'acide nucléique de SEQ ID N° 1 ;
b) les séquences d'ARNm et d'ADNc correspondantes.

La séquence [SEQ ID N°2] est la séquence d'acides aminés déduite de la séquence [SEQ ID N°1].

La séquence d'ADN [SEQ ID N°1] peut être obtenue à partir de la séquence d'ADN HindIII-HindIII de 3,3 kb contenue dans la souche Lactococcus lactis ssp lactis déposée à la C.N.C.M. sous le N°I-945 par la méthode PCR à l'aide de deux oligonucléotides ci-après :

L'invention a également pour objet les plasmides transformés avec l'une des séquences d'acides nucléiques selon l'invention. Ces plasmides peuvent être par exemple le plasmide pPF144-12, dans lequel on a cloné, selon les techniques habituelles bien connues de l'homme de l'art, la séquence d'ADN selon l'invention.

L'invention a également trait aux bactéries lactiques résistantes aux phages, de préférence appartenant à l'espèce Lactococcus lactis, qui contiennent au moins une séquence d'acides nucléiques ou un plasmide tel que défini ci-dessus.

Cette séquence d'acides nucléiques ou ce plasmide peuvent avoir été introduits dans les bactéries lactiques par conjugaison, transformation, fusion de protoplastes ou par une autre méthode de transfert de gènes.

Les bactéries lactiques qui peuvent avantageusement être transformées à l'aide de la séquence d'acides nucléiques selon l'invention ou d'un plasmide la contenant sont par exemple les souches *Lactococcus lactis ssp cremoris, Lactococcus lactis ssp lactis, Lactococcus lactis ssp lactis var.diacetylous.*

Ces souches ainsi transformées peuvent être utilisées pour transmettre par conjugaison, transformation, transduction, fusion de protoplastes ou une autre méthode de transfert de gènes, un mécanisme de résistance aux phages à une souche d'intérêt industriel. Ce mécanisme peut être porté par un plasmide ou par une autre partie du génome de la bactérie. Lorsque celui-là est porté par un plasmide il est avantageux de le transférer par conjugaison.

L'invention a également trait aux souches d'intérêt industriel résistantes aux phages ainsi obtenues.

L'invention sera mieux comprise à l'aide des exemples ci-après, qui comprennent des résultats expérimentaux et une discussion de ceux-ci. Certains de ces exemples concernent des expériences effectuées dans le but de réaliser l'invention, d'autres des exemples de réalisation de l'invention sont donnés bien sûr à titre purement illustratif.

Une grande partie de l'ensemble des techniques décrites dans ces exemples, bien connues de l'homme de l'art, est exposée en détail dans l'ouvrage de Sambrook Fritsch et Maniatis : "Molecular cloning ; a Laboratory Manual" publié en 1989 par les éditions Cold Spring Harbor Press à New York (2ème édition).

La description ci-après sera mieux comprise à l'aide des figures 1 à 2 ci-après qui représentent respectivement :

FIG. 1 : Carte de restriction du fragment 3,3 kb de pPF 144-1.

FIG. 2 : Amplification par PCR de fragments internes du fragment 3,3 kb de pPF 144-1.

Le fragment 1-2 confère la résistance aux phages.

Les fragments 1-4, 2-3 et 3-4 ne confèrent pas la résistance aux phages.

### Exemple 1 : Séquence du fragment HindIII-HindIII de 3,3 kb

La souche S45-91-1 de *Lactococcus lactis* déposée à la C.N.CM sous le N°I-945 le 12 avril 1990, contient un plasmide d'une taille de 144 kb nommé pPF144, qui confère la résistance aux phages. Cette souche est totalement résistante au phage ∅59 (groupe III). Par contre, elle présente une résistance partielle contre le phage ∅53 du groupe I, qui se développe mais en formant des plages de lyse anormalement petites, de la taille d'une tête d'épingle. Le fragment de restriction HindIII-HindIII de 3,3 kb, conférant la résistance aux phages, a été cloné à partir du plasmide pPF144 dans le vecteur pVA838 décrit par MACRINA F.L. et al. (1982), Gène, 19, 345-353, selon le mode opératoire décrit à l'exemple 7 de EP-A1-452 224, incorporé à la présente à titre de référence. Ce plasmide recombinant, pPF144-2, confère à la souche *Lactococcus lactis ssp lactis S56* le même niveau de résistance aux phages que le plasmide pPF144 en entier.

La séquence d'acides nucléiques de ce fragment de 3,3 kb déterminée par la méthode de Sanger et al (PNAS-USA, 14, 5463, 1977) est la séquence [SEQ ID No.7] ci-après.

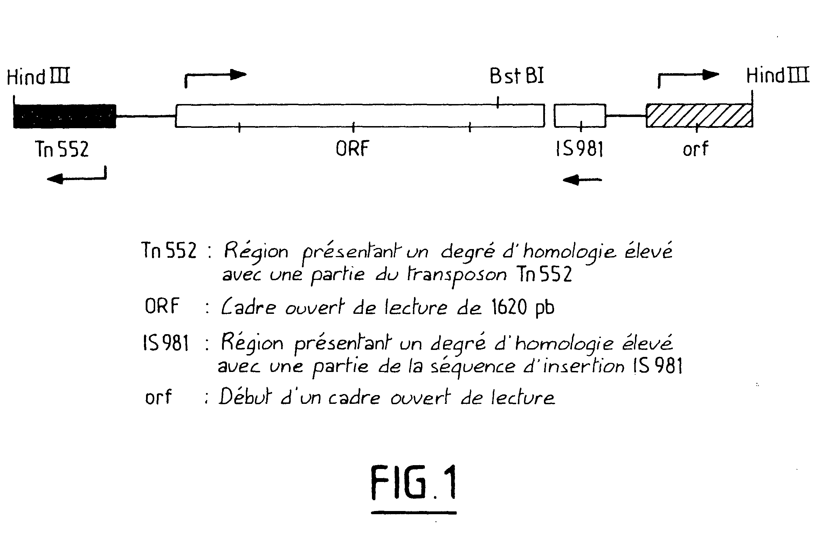

Des analyses par restriction enzymatique de ce fragment de 3,3 kb ont montré la présence dans ce fragment d'un site unique de reconnaissance de l'enzyme BstBI. Le sous-clonage des deux fragments de restriction HindIII-BstBI et leur introduction dans la souche S56 de *L.lactis*, ont permis de montrer qu'aucun des deux ne confère la résistance aux phages. Il en a été déduit que le site BstBI est à l'intérieur du gène de résistance supposé. Cette hypothèse a été confortée par la détermination de la séquence nucléotidique des deux fragments, montrant que le site BstBI est à l'intérieur d'un cadre ouvert de lecture (ORF) de 1,62 kb, qui correspondrait au gène de résistance. Sur la figure 1 on a représenté la carte de restriction du fragment HindIII-HindIII de 3,3 kb.

D'autres analyses ont également montré que le fragment HindIII-HindIII de 3,3 kb possède :
- une région Tn 552 présentant un degré d'homologie élevé avec une partie du transposon Tn 552 (réf : Tn 552, a novel transposable element from Staphylococcus aureus (1990). S.J ROWLAND, K.G.H. DYKE. Molecular Microbiology 4 : 961-975).
- un cadre de lecture ORF de 1620 pb qui correspondrait au gène de résistance ;
- une région IS 981 présentant un degré d'homologie élevé avec une partie de la séquence d'insertion IS 981 (réf : Identification, DNA sequence, and distribution of IS 981, a new high-copy-number insertion sequence in Lactococci (1991) K.M. POLZIN, L.L McKAY Applied and Environ. Microbiol. 57: 734-743.
- le début d'un cadre ouvert de lecture (orf).

### Exemple 2 : Amplification par PCR de fragments internes du fragment HindIII-HindIII de 3,3 kb

La technique de la "PCR" (Polymerase Chain reaction), décrite par exemple dans l'ouvrage de Maniatis, déjà cité, permet d'amplifier un fragment d'ADN compris entre deux oligonucléotides. Cet ADN amplifié peut être aisément cloné si des sites de restriction sont apportés par les oligonucléotides. En effet, les séquences de ces oligonucléotides peuvent comporter, à leur extrémité 5', une partie hétérologue de l'ADN à amplifier, constituée par exemple de 8 paires de bases, dont 6 constituent un site de restriction.

Cette technique a été appliquée en vue de déterminer si l'ORF mise en évidence dans la séquence nucléotidique du fragment de 3,3 kb correspondait bien au gène de résistance aux phages, mais également en vue de constituer une sonde spécifique de cet ORF.

4 oligonucléotides de 28 bases (dont 6 constituent un site de restriction) ont été synthétisés.

La séquence de ces 4 oligonucléotides est la suivante :

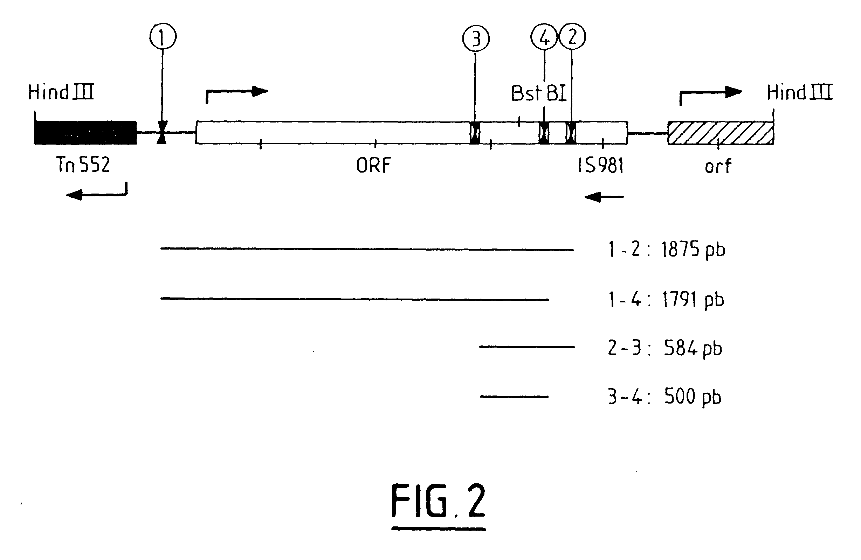

Leurs emplacements sur le fragment de 3,3 kb sont indiqués sur la figure 2.

Les oligonucléotides N° 1 et 2 ont permis d'amplifier un fragment d'ADN de 1875 pb comportant l'ORF entière plus 201 pb directement en amont de celle-ci, région susceptible de contenir des signaux d'expression du gène. Cet ADN a été amplifié sous la forme d'un fragment EcoRI-BamHI grâce aux sites de restriction apportés par les oligonucléotides, permettant un clonage directionnel dans le vecteur navette pVA838.

De la même manière, les oligonucléotides N° 3 et 4 ont permis d'amplifier une région de 500 pb, chevauchant le site BstBI, sous forme d'un fragment EcoRI.-EcoRI. Cette région a été choisie pour constituer une sonde spécifique car il a été montré que les deux sous-fragments HindIII-BstBI du fragment de 3,3 kb ne conféraient pas à eux seuls la résistance aux phages et donc que la région du site BstBI était essentielle à l'activité du gène.

Deux autres fragments internes à l'ORF ont pu être amplifiés par "PCR" grâce aux paires d'oligonucléotides N° 1 et 4 et N° 2 et 3.

Les 4 fragments d'ADN ont été amplifiés par "PCR" à partir du plasmide pPF144-2 purifié sur CsCl, avec la Vent polymérase (Biolabs) qui possède une activité exonucléase, augmentant sa fidélité d'un facteur 15 par rapport à la Taq polymérase classique. Les produits de PCR ont été purifiés par une extraction au phénol-chloroforme, précipités à l'éthanol, digérés par EcoRI ou BamHI et EcoRI suivant le fragment et clonés dans le vecteur pVA 838.

Le clonage des fragments dans le vecteur pVA 838 a permis de les introduire, après amplification des plasmides recombinants dans la souche TG1 d'*E.coli*, dans une souche de *L. lactis* et de déterminer s'ils confèrent la résistance aux phages.

Une synthèse des résultats concernant le clonage des différents fragments d'ADN amplifiés est présentée dans le tableau I suivant :

**TABLEAU I**

| paire d'oligonucléotides | taille du fragment | sites rajoutés | cloné dans pVA838 |
|---|---|---|---|
| 1-2 | 1875 pb | EcoRI-BamHI | pPF144-12 |
| 1-4 | 1791pb | EcoRI-EcoRI | pPF144-14 |
| 2-3 | 584 pb | BamHI-EcoRI | pPF144-23 |
| 4-3 | 500 pb | EcoRI-EcoRI | pPF144-43 |

### Exemple 3 : Résistance aux phages conférée par le plasmide pPF144-12,

Les plasmides pPF144-12, pPF144-14, pPF144-23 et pPF144-43 ont été introduits dans la souche S56 de *L.lactis*. La résistance aux phages des clones obtenus a été testée en réalisant une titration (UFP/ml) avec les phages ∅53 et ∅59.

Les résultats sont donnés ci-après :

| Souche | phage ⌀53 (I) | | phage ⌀59 (III) | |
|---|---|---|---|---|
| | Titre (UFP/ml) | Taille des plages (mm) | Titre (UFP/ml) | Taille des plages (mm) |
| S56 | 10¹⁰ | 3 | 3.10⁹ | 2 |
| S56(pPF144-1) | 2.10⁷ | < 0,25 | 0 | 0 |
| S56(pPF144-12) | 4.10⁷ | < 0,25 | 0 | 0 |
| S56(pPF144-14) | 8.10⁹ | 3 | 6.10⁹ | 2 |
| S56(pPF144-23) | 6.10⁹ | 3 | 6.10⁹ | 2 |
| S56(pPF144-43) | 10¹⁰ | 3 | 2.10⁹ | 2 |
| UFP/ml = unité formant plages par ml. | | | | |

Le plasmide pPF144-12, contenant le fragment amplifié par PCR de 1875 pb, confère la même résistance aux phages que le plasmide pPF 144-1. Les autres plasmides, pPF 144-14, pPF 144-23 et pPF 144-43, ne comprenant qu'une partie de l'ORF de 1,62 kb, ne confèrent pas la résistance aux phages

### Exemple 4 : Test sur la réplication de l'ADN phagique en présence du plasmide pPF144-12

Les phages ∅53 et ∅59 appartiennent, respectivement, aux groupes génétiques I et III. Une carte génétique de ces phages a été construite et il a surtout été montré que le génome de ces phages, constitué d'ADN double-brin, possède des extrémités cohésives. Ce résultat implique que la réplication de l'ADN de ces phages se déroule selon un modèle identique à celui du phage Lambda d'E. coli : formation de concatémères au cours du cycle lytique et découpage de ces concatémères par une enzyme spécifique lors de l'encapsidation dans la nucléocapside du phage.

La méthode de Hill et Coll. (Hill, C. Massey, I.J., Klaenhammer, T.R. (1991). Rapid method to characterize lactococcal bacteriophage genomes. Appl. Environ. Microbiol. 57 : 283-288) a été utilisée pour suivre le devenir de l'ADN phagique après injection dans la bactérie. La souche de L. lactis S56 contenant le vecteur pVA838 ou le plasmide pPF144-12 a été infectée avec ∅53 et ∅59 à une multiplicité d'infection de 2. Des parties aliquotes des cultures infectées sont prélevées à intervalles de temps réguliers. L'ADN total, cellulaire et phagique, de chaque partie aliquote est extrait, digéré par une enzyme de restriction et les fragments obtenus sont séparés par une migration en gel d'agarose par électrophorèse. L'ADN est ensuite transféré sur membrane de nylon et soumis à une hybridation avec l'ADN du phage utilisé comme sonde (kit ECL, Amersham).

Cette méthode permet de suivre l'apparition et l'évolution de l'ADN phagique à l'intérieur de la cellule infectée en fonction du temps.

Les résultats obtenus avec les enzymes EcoRI, HindIII et EcoRV ont montré que l'ADN phagique se réplique dans les souches S56 avec le vecteur pVA838 ou le plasmide pPF144-12. On observe une accumulation de l'ADN phagique sous forme de concatémères avec le plasmide pPF144-12 alors que dans la souche contenant le vecteur pVA838, ces concatémères commencent à disparaître dès la vingtième minute après l'infection.

### Exemple 5 : Test sur la production de protéines phagiques en présence du plasmide pPF144-12

Des préparations purifiées sur chlorure de Césium des phages ⌀53 et ∅59 ont servi à préparer des anticorps polyclonaux chez le lapin. La souche S56 contenant le vecteur pVA838 (témoin) ou le plasmide pPF144-12 a été infectée par l'un de ces phages à une multiplicité de 1. Toutes les cinq minutes après infection, une fraction des cellules est prélevée, chauffée trois minutes à 100°C en présence de 2,3 % SDS et 5 % β-mercaptoéthanol, et les protéines sont fractionnées sur un gel SDS-polyacrylamide 12,5 % [Laemmli, U.K. 1970. Cleavage of structural proteins during the assembly of the head of bacteriophage T4. NATURE (London) 227 : 680-685], puis transférées sur des feuilles de nitrocellulose.

La détection immunologique des protéines phagiques sur la nitrocellulose a été effectuée avec des anticorps de lapin anti-⌀53 ou ⌀59 puis le complexe immunologique a été localisé avec des anticorps de souris anti-lapin (kit ECL Amersham) avec la streptavidine-phosphatase alcaline.

Ces résultats montrent que l'on trouve les protéines de phages ⌀53 ou ⌀59 avec et sans pPF144-12, mais qu'en présence de ce plasmide, la quantité de protéines produite est faible, et la vitesse d'apparition de ces protéines est ralentie par rapport à une souche contenant le vecteur pVA838. Ce phénomène est plus marqué pour ⌀59 que pour ⌀53.

## Revendications

1. Séquence d'acides nucléiques comprenant au moins un mécanisme de résistance aux phages, **caractérisée en ce qu'**elle est constituée par :
a) la séquence d'ADN présentant l'enchaînement d'acides nucléiques de SEQ ID N° 1 ;
b) les séquences d'ARNm et d'ADNc correspondantes.

2. Plasmide comprenant au moins un mécanisme de résistance aux phages **caractérisé en ce qu'**il comporte une séquence d'acides nucléiques selon la revendication 1.

3. Bactérie lactique résistante aux phages, **caractérisée en ce qu'**elle contient au moins un plasmide selon la revendication 2.

4. Utilisation des bactéries lactiques selon la revendication 3, pour conférer un mécanisme de résistance aux phages à des souches d'intérêt industriel.

## Patentansprüche

1. Nucleinsäuresequenz, die mindestens einen Resistenzmechanismus gegen Phagen umfaßt, **dadurch gekennzeichnet, daß** sie besteht aus:
a) der DNA-Sequenz, die die Nucleinsäure-Verknüpfung von SEQ ID Nr. 1 aufweist;
b) den entsprechenden mRNA- und cDNA-Sequenzen.

2. Plasmid, das mindestens einen Resistenzmechanismus gegen Phagen umfaßt, **dadurch gekennzeichnet, daß** es eine Nucleinsäuresequenz nach Anspruch 1 enthält.

3. Milchsäurebakterie, die gegen Phagen resistent ist, **dadurch gekennzeichnet, daß** sie mindestens ein Plasmid nach Anspruch 2 enthält.

4. Verwendung der Milchsäurebakterien nach Anspruch 3, um Stämmen von industriellem Interesse einen Resistenzmechanismus gegen Phagen zu verleihen.

## Claims

1. A nucleic acid sequence comprising at least one phage resistance mechanism, said sequence consisting of:
a) the DNA sequence having the nucleic acid series of SEQ ID no. 1 ;
b) the corresponding mRNA and cDNA sequences.

2. A plasmid comprising at least one phage resistance mechanism, said plasmid containing a nucleic acid sequence according to claim 1.

3. A phage-resistant lactic acid bacterium which contains at least one plasmid according to claim 2.

4. Use of the lactic acid bacteria according to claim 3 for conferring a phage resistance mechanism on strains of industrial interest.
